# EUROPEAN PATENT APPLICATION

(11) **EP 1 142 906 A1**
(43) Date of publication of application: **10.10.2001**
(21) Application number: 01303073.9
(22) Date of filing: 30.03.2001
(51) Int. Cl.: C07K 14/08, A61K 39/42, C07K 16/10, G01N 33/576

(54) **A hepatitis B virus (subtype ayw) surface antigen variant**

(30) Priority: 31.03.2000 US 193795 P
(71) Applicant: Ortho-Clinical Diagnostics, Inc., Rochester, NY 14626-5101 (US)
(72) Inventor: Zheng, Jian, Raritan, NJ 08869 (US)
(74) Representative: Mercer, Christopher Paul

(57) **Abstract**

We isolated and characterized a new surface mutant of the hepatitis B virus surface antigen (HBsAg). The mutant was isolated from a symptomatic patient with Down's syndrome who was found to be persistently positive for both for HBsAg and anti-HBs Antibody (Ab) with an equally long-lasting anti-HB core (c) IgM Ab

## Description

Hepatitis B virus (HBV), a small double stranded DNA virus, can cause a wide spectrum of clinical presentations: asymptomatic carrier state, acute self-limited hepatitis, fulminant hepatitis, and chronic liver diseases including chronic hepatitis, liver cirrhosis, and hepatocellular carcinoma. It has a circular genome of 3182 to 3221 base pairs (bp). Four major subtypes have been identified and can be differentiated by antibodies that recognize the different epitopes on the HBV surface. The HBsAg particles carry the common determinant, "a", as well as d or y and w or r subtype determinants, and are classified into the four major subtypes, i.e., adw, adr, ayw and ayr. Rare sera contain HBsAg particles with all four-subtype determinants (adywr). The antigenic determinants for the main HBV subtypes: adw, adr, ayw and ayr lie in the surface or "S" polypeptide. Two amino acid residues in particular, encoded by the S gene at codon positions 122 and 160, have been postulated to determine the different antigenic subtypes. While, the preC regions have frequently been reported to have mutations rendering HBe Ag negative. The virus has a high rate of mutation relative to other DNA viruses due to its mode of replication by reverse transcriptase of its pregenomic RNA. The importance of a novel mutant can be reflected in vaccine escape and HBsAg detection failure, implicating a public health problem.

### Summary of the Invention

We have identified and characterized a new surface mutant of HBV. The mutant was isolated from a symptomatic patient with Down's syndrome who was found to be persistently positive for both for HBsAg and anti-HBs Antibody (Ab) with an equally long-lasting anti-HB core (c) IgM Ab. With a panel of six monoclonal antibodies (mAb(s)) to HBsAg, we evaluated the mutation influence on the major epitope of the "a" determinant antigenicity.

### Brief Description of the Drawings

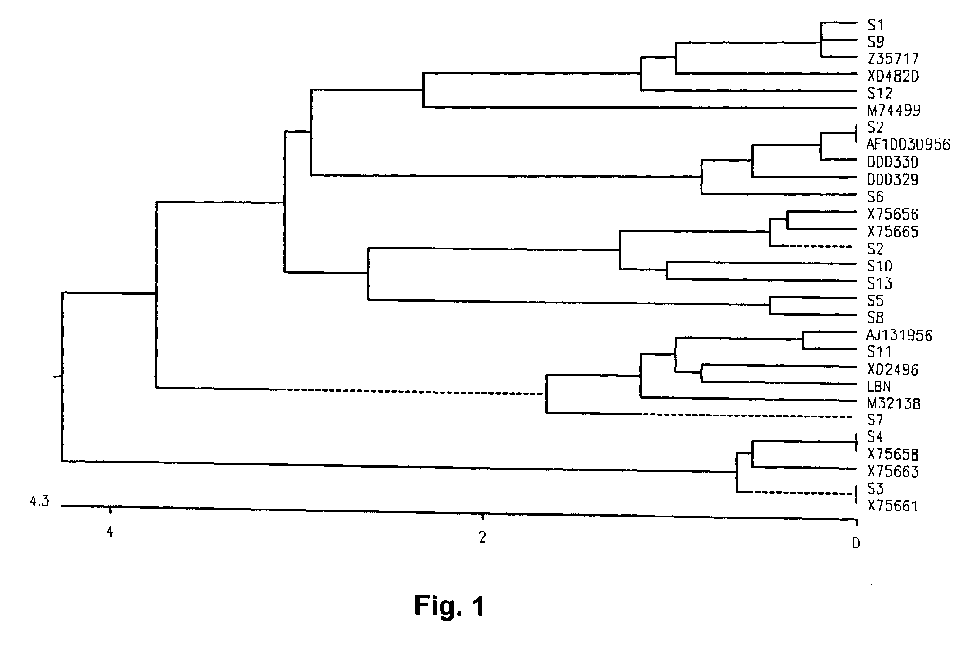

Figure 1 illustrates the phylogeny tree of the HBsAg genomes and the accession numbers. Sequence of S1 - S13 strains are obtained from reference (Norder H, Hammas B, Lofdahl S, Courouce AM, Magnius LO. Comparison of the amino acid sequences of nine different serotypes of hepatitis B surface antigen and genomic classification of the corresponding hepatitis B virus strains. J Gen Virol. 1992 May;73 ( Pt 5):1201-8); GenBank accession numbers are given for the rest of strains.

### Detailed Description

A sample was taken from a 43-year-old white male patient with Down's syndrome who presented with jaundice to the American University of Beirut Medical Center in March 1999. The patient had no history of immunization to HBV, no transmissible risk factor including blood transfusion, IV drugs, homosexuality, family HBV infection or hemodialysis. During investigation, he was found to have elevated liver function tests (LFT): ALT 450 IU/L (range 10-35 IU/L), AST 250 IU/L (range 10-40 IU/L), gamma-GT 383 IU/L (range 10-50 IU/L), Bilirubin was 3.8/2.6 mg/dL (total/direct) (range, total: 0.1-1.2 IU/L and direct: 0.0-0.2 IU/L). More significantly, we noted incongruity in the HBV blood tests. The HBV markers test results using enzyme immunoassay (EIA) methodology were as follows: HBs Ag positive (>2/0.051), anti-HBs Ab positive (0.417/0.206), anti-HBc IgM Ab low positive (0.371/0.208), anti-HBc IgG Ab strong positive (>2/0.412), HBe Ag negative and anti-HBe Ab positive (0.197/0.830, a competitive assay). Both the anti-hepatitis A Ab and the anti-hepatitis C Ab were negative. Due to the discrepancy in the HBV markers, molecular methods were used to confirm the HBV antigen. The patient was continuously followed up for LFT that decreased consistently over the following ten months including AST 80 IU/L. However, the HBV markers that were repeated ten months after initial presentation revealed both HBsAg positive (>2/0,056) (DiaSorin, Italy) and anti HBs Ab positive (56/5) (BioMerieux, Mini-Vidas, France), and the persistence of a low positive anti-HBc IgM Ab (0.303/0.221) (DiaSorin, Italy).

### Example 1: Extraction and Amplification of HBs DNA

DNA was isolated and purified from 40 uL samples based on a guanidinium-thiocynate (GuSCN) lysis method. The single-step method of RNA isolation by acid guanidinium thiocyanate-phenol-chloroform extraction was used with some modifications. Five volumes of lysis solution [5.75 M GuSCN, 50 mM Tris-Cl pH7.5, 100 mM 2-mercaptoethanol and 15 ug/mL poly (A)] were added to 1 volume of serum. After a brief vortex, the mixture was heated at 60°C for 10 minutes. The DNA pellet was obtained with isopropanol precipitation, and was washed with 75% ethanol. Dried DNA pellet was then resuspended in 50 uL of PCR reaction solution, with 40 uM (each) of the first-round primers (F1-6 and R1-6). The mixture was heated to 95°C for 3 min, followed by 30 PCR cycles consisting of 94°C/45 sec, 55°C/45 sec and 72°C/45 sec in a thermal cycler (GeneAmp PCR System 9600, Perkin Elmer, USA). 0.5 uL of the first-round PCR product was then served as template for a second-round PCR amplification using F1-7 and R13-2d primers pair consisting same cycles except annealing temperature was raised to 60°C. Positive PCR products, a DNA band of 604bp as expected, were detected in agarose gel electrophoresis.

The PCR Primer sequences [nucleotide sequences were derived from HBV DNA (accession number J02203) were as follows:
First-round Primer Set: F1-6 (231-252): CCTCACAATACCGCAGAGTCTA R1-6 (816-842:                    AGGGTTTAAATGTATACCCAAAGACAA
Second-round Primer Set:

F1-7 (246-268): GAGTCTAGACTCGTGGTGGACTT

R13-2d (822-843):    ccgaattcTAGGGTTTAAATGTATACCCA

An EcoR I site was added at the 5'-end of R13-2d for cloning purpose.

### Example 2: Seguence Alignment and Phylogenetic Analysis

Amplified PCR product was cleaned with a QIAquick spin column (Qiagen) and subsequently cloned for DNA sequencing and protein expression. Five clones were sent for DNA sequencing in order to obtain reliable DNA sequence determination. Nucleotide sequences were determined for both strands with the BigDye Terminator Ready Reaction Kit (PE Applied Biosystems, USA) on an ABI 377 DNA Sequencer (PE Applied Biosystems, USA). Sequence analysis was performed using SeqMan 4.00 module of the Lasergene package (DNAStar Inc., Madison, WI, USA). Sequence alignment and the construction of phylogenetic trees were computed by MegAlign 4.00 module of the Lasergene package (DNAStar Inc., Madison, WI, USA). Clustal multiple sequence alignment was used through sequence weighting. The 28 reference strains for genotype grouping were derived from published sequences.

### Example 3: S-HBsAg Cloning and Transient Protein Expression

The PCR product was digested by Xba I /EcoR I restriction enzymes. The 595 bp fragment encoded 86% of the S-HBsAg protein from amino acids Leu 32 to the end (Ileu 226). The fragment was then ligated into a previously constructed mammalian expression vector, to replace the wild-type ayw S-HBsAg fragment, which was placed downstream of a CMV promoter. The transfection was then performed on a COS-7 cell line using LIPOFECTAMINE Plus reagent (Life technologies, MD, USA). Culture supernatant containing secreted variant S-HBsAg from the COS-7 cell infections was then harvested and fresh medium was added every 72 hours after transfection. Wild-type ayw [wt(ayw)] S-HBsAg, (GeneBank accession number J02203), was also expressed for control use.

### Example 4: Recombinant HBsAg Antigen Immunoassay and Epitope Analysis

Recombinant wt(ayw) and LBN variant S-HBsAg expression was determined by solid-phase EIA in a sandwich format. One polyclonal and a panel of six monoclonal antibodies [mAb(s)] were used in this study. They were goat anti-HBsAg (*ay/ad*) (Fitzgerald, MA, USA), HB-1, HB-8, HB-9, HB-13, HB-14 and HB-16. All six mAb(s) were raised against serum derived wild-type HBsAg and were reactive to both ad and ay subtypes. EIA were used to measure serum and recombinant HBsAg reactivity. Basically, purified mAb(s) or polyclonal anti-HBsAg were coated on a microtiter plate and kept at 4°C until use. Prior to performing EIA, the plates were blocked with PBS containing BSA for 2 hours at 25°C. EIA was performed in a one-step sandwich format with 150 uL of sample (serum or diluted recombinant HBsAg culture) mixed with 50 uL HRP conjugated monoclonal antibodies. The plates were incubated at 37°C for 90 minutes, then washed 6 times with PBS containing 0.005% Tween 20. The plates were developed by a final incubation for 30 minutes with 200 ul of OPD solution (Sigma, USA). Adding 50 uL of 4N Sulfuric acid then stopped the color development. The absorbance was read on an automatic microtiter plate reader (Molecular Devices, USA) and the results were expressed as optical density (OD) units at 490 nm. To evaluate the mAb reactivity to the native HBsAg and to the synthetic peptides, direct EIA procedure was performed. A serial dilution of each purified and quantity adjustified mAb was incubated on plates, which were previously coated with purified native HBsAg or oxidized peptides. Bound murine IgG were detected by a second incubation with horseradish peroxidase conjugated rabbit anti-mouse IgG. The reactivity was ascertained by enzyme catalyzed OPD color development. Recombinant variant and wild-type S-HBsAg were also analyzed by western immunoblot. HBsAg culture supernatant was concentrated 15 fold by a centrifugal filter (Biomax-100, Millipore, USA). The concentrate was run on a NuPAGE 10% Bis-Tris SDS Gel (Novex, San Diego, USA) and then transferred to nitrocellulose membrane. Transferred protein was immunoblotted with HBsAg mAb. The binding was detected using an immunoblot system (BioRad, GAM-AP, USA).

### Example 5: HBs Variant DNA Sequence and Amino Acid Sequence Analysis

The HBs DNA sequence of this novel Lebanese (LBN) variant was analyzed for genotypes. Sequence comparison between variant HBs DNA sequence and other 28 defined HBs genotype strain put this variant HBs into genotype D group (Fig. 1). Although serological typing on this patient's serum was not performed, the amino acid sequence of this HBs variant showed highest homology with ayw2 HBs subtype. Five amino acid mutations within position 32-226 of LBN variant were observed in all five clones. They are 103 M (ATG)→ I (ATT) , 118 T (ACG)→ K (AAG), 120 P (CCA) → Q (CAA), 170 L (TTA)→ S (TCA) and 213 L (TTA) → S (TCA). At amino acid position 122, three clones revealed sequence Arg (AGA) while 2 clones revealed sequence as Lys (AAA). ). For recombinant LBN HBsAg expression, sequence with Arg (122) was used.

102 - 110 - 120 - 130 - 140 - 150
wt(ayw2) GMLPVCPLIPGSSTTSTGPCRTCTTPAQGTSMYPSCCCTKPSDGNCTCI
LBN -I--------------K-Q------------------------------

Wild-type HBsAg consensus sequence was derived from other three strains (X02496, M32138 and S7) of the genotype D group. Bold indicates the "a" determinant region (Stirk HJ, Thornton JM, Howard CR. A topological model for hepatitis B surface antigen. Intervirology. 1992;33(3) :148-58)

### Example 6: Recombinant HBsAg Transient Expression in COS-7 Cell

The secreted recombinant HBsAg expression was in a range of 0.1-0.2 mg/liter, calibrated by a purified wild-type recombinant HBsAg. Only secreted HBsAg in culture supernatant was examined for expression. There was no significant expression yield difference at 72 hours and 144 hours time point. The culture supernatant harvested at 216 hours gave only 1/5 expressed HBsAg compared to the supernatants collected at 72 and 144 hours.

### Example 7: Immunoreactivity Analysis:

Both the recombinant HBsAg (ayw) and the novel variant HBsAg (LBN) culture supernatants were tested for their reactivity by a panel consisting of six mAb(s). Culture supernatants were diluted 1:40 to insure the same quantity of antigen concentration. Amino acid mutations apparently affected four mAb(s) binding affinity to wt HBsAg. HB-16 was affected most, losing almost 90% strength. Strikingly, the binding strength of two mAb(s), HB-13 and HB-14, to the LBN variant was significantly increased to 5-6 fold. Peptide mapping revealed that these two antibodies were specific to the first loop linear sequence while mAb HB-9 was specific to the sequence from 137 to147, located in the second loop region. All the three mAb(s), HB-1, HB-8 and HB-16, are conformation specific. They did not react with SDS denatured HBsAg in immunoblot but had higher dilution titer in direct EIA assay. On the other hand, the mAb(s) HB-13 and HB-14 blotted to both wild type and LBN HBsAg very well, indicating that these two mAb(s) recognize denatured epitopes, independent of the mutation sites.

**Table 1.**

| Immunal reactivity of a panel of monoclonal antibodies to various antigens in EIA assay and in western immunoblot | | | | | | | | | | | | | |
|---|---|---|---|---|---|---|---|---|---|---|---|---|---|
| | | Monoclonal antibodies raised against wild type HBsAg | | | | | | | | | | | |
| | | ***HB-1*** | | ***HB-8*** | | ***HB-9*** | | ***HB-13*** | | ***NB-14*** | | ***HB-16*** | |
| mAb relative reactivity (%) to LBN variant * | | 55.5 | | 55.7 | | 35.3 | | 530.1 | | 665.2 | | 10.3 | |
| | | | | | | | | | | | | | |
| mAb titer against native HBsAg ** | | 1:8X10⁵ | | 1:2X10⁵ | | 1:2X10⁴ | | 1:4X10⁴ | | 1:4X10⁴ | | 1:4X10⁵ | |
| | | | | | | | | | | | | | |
| peptide (122-137)# | | 0.161 | *1:50* | 0.154 | *1:50* | 0.125 | *1:50* | >2.00 | *1:2K* | >2.00 | *1:500* | 0.121 | *1:50* |
| peptide (139-147)# | | 0.183 | *1:50* | 0.223 | *1:50* | >2.00 | *1:500* | 0.250 | *1:50* | 0.331 | *1:50* | 0.119 | *1:50* |
| peptide (122-147)# | | 0.201 | *1:50* | 0.261 | *1:50* | >2.00 | *1:2K* | 0.292 | *1:50* | 0.340 | *1:50* | 0.143 | *1:50* |
| | | | | | | | | | | | | | |
| Western | wt | - | | - | | n.p. | | ++ | | +++ | | - | |
| Immunoblot | (ayw) LBN | - | | - | | n.p. | | ++ | | +++ | | | |

| | | | | | | | | | | | | | |
|---|---|---|---|---|---|---|---|---|---|---|---|---|---|
| * Relative reactivity was presented as OD derived from each mAb reactivity to LBN variant compared with OD derived from its reactivity to wt(ayw) which was set at 100%. | | | | | | | | | | | | | |
| ** mAb titer was expressed as dilution needed for each mAb to obtain a preset signal between OD=1.00 to 1.25 in a direct EIA with native HBsAg. | | | | | | | | | | | | | |
| # Reactivity was expressed as OD obtained from each mAb at indicated dilution. All mAb(s) concentration was adjusted at a same concentration start point. All peptide sequence was based on wt(ayw). n.p. : not performed. Western immunoblot results were visually determined. | | | | | | | | | | | | | |

## Claims

1. An isolated variant hepatitis B surface antigen comprising an amino acid sequence wherein mutations from hepatitis B wild type ayw2 strain appear as follows: at position 103 isoleucine is present instead of methionine, at position 118 lysine is present instead of threonine, at position 120 glutamine is present instead of proline, at position 170 serine is present instead of leucine, and at position 213 serine is present instead of leucine.

2. An expression vector for expression of a variant hepatitis B surface antigen in a recombinant host, wherein said vector contains a recombinant gene encoding the variant hepatitis B surface antigen of claim 1.

3. A monoclonal antibody raised against the variant hepatitis B surface antigen of claim 1.

4. A hybridoma cell line which secretes the monoclonal antibody of claim 3.

5. An assay kit for determining the presence of hepatitis B in a test sample, comprising: a container containing at least one monoclonal antibody which specifically binds to hepatitis B surface antigen wherein the monoclonal antibody is a monoclonal antibody secreted by the hybridoma cell line claimed in claim 4.

6. A method for determining the presence of hepatitis B in a test sample, comprising:
a. contacting a test sample with at least one monoclonal antibody claimed in claim 3 attached to a solid phase, to form a mixture,
b. incubating the mixture for a time and under conditions sufficient to form antigen-antibody complexes,
c. contacting the complexes with an antibody conjugated to a signal generating reagent that is specific for the complexes, and
d. determining the presence of hepatitis B present in the test sample by detecting the signal generated.
